# DEMANDE DE BREVET EUROPEEN

(11) **EP 4 609 937 A1**
(43) Date de publication de la demande: **03.09.2025**
(21) Numéro de dépôt: 25153673.6
(22) Date de dépôt: 23.01.2025
(51) Int. Cl.: B01D 53/04, A61M 11/00, B01D 53/32, C25B 1/02, A61M 16/08, A61M 16/10, A61M 16/20, A61M 16/22

(54) **INSTALLATION DE RÉGÉNÉRATION ET REMPLISSAGE D'UNE CARTOUCHE D'OXYGÈNE**

(30) Priorité: 12.02.2024 FR 2401359
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, 75007 Paris (FR)
(74) Mandataire: Air Liquide

(57) **Abrégé**

L'invention concerne une installation (50) de conditionnement d'une cartouche d'O₂ (5) comprenant une unité de production d'oxygène (3) coopérant avec une unité de remplissage (1) comprenant un dispositif de distribution de gaz (100), des moyens de réception de cartouche (140) pour recevoir une cartouche d'O₂ (5) munie de deux valves (51, 52) contrôlant les flux gazeux entrant et sortant de la cartouche d'O₂ (5), et un port de liaison à l'atmosphère (123a) en communication fluidique avec l'atmosphère ambiante (A).

## Description

La présente invention concerne une installation ou station de conditionnement, i.e. de régénération et de remplissage, d'une cartouche d'oxygène équipée de deux valves de contrôle des entrées et sorties de gaz, et contenant un matériau adsorbant sélectif du CO₂.

EP4227575 enseigne une cartouche d'oxygène de petite taille ayant un volume interne inférieur à 1L (équiv. en eau) contenant un adsorbant de type zéolite. Elle contient un volume d'oxygène (O₂) de plusieurs litres, par exemple 15 L, à basse pression (i.e. < 10 bar), pouvant être déchargé dans un réservoir flexible, après activation, c'est-à-dire lorsqu'elle est utilisée.

En utilisation, la cartouche est couplée à une interface respiratoire de sorte qu'un utilisateur peut respirer l'oxygène qui circule à travers la cartouche pendant ses phases inspiratoire. Pendant ses phases expiratoires, le dioxyde de carbone (CO₂) contenu dans les gaz expirés par l'utilisateur est piégé par l'adsorbant, alors que l'O2 non métabolisé se trouvant dans les gaz expirés n'est pas retenu.

Les gaz expirés sont donc purifiés du CO₂ qu'ils contiennent, voire d'une partie de la vapeur d'eau pouvant s'y trouver, mais pas de l'oxygène et peuvent être alors réinhalés par l'utilisateur.

Autrement dit, un tel système permet de recycler l'oxygène se trouvant dans les gaz expirés par l'utilisateur, c'est-à-dire à éliminer le CO₂ tout en conservant l'oxygène qui s'y trouve, ce qui offre une autonomie accrue, i.e. une durée d'utilisation, et évite de gaspiller l'oxygène expiré qui peut représenter jusqu'à 95% du flux de gaz expirés OK.

De telles cartouches sont donc bien adaptées, du fait de leur petite taille, de leur légèreté, de leur autonomie relativement importante, à une utilisation notamment par les personnels soignants militaires ou analogues, notamment sur les terrains d'opération militaires (e.g. OPEX), les zone de combat ou analogues.

Toutefois, une fois utilisée, cette cartouche nécessite d'être recyclée, c'est-à-dire régénérée et remplie à nouveau. Ceci peut être fait dans un centre de remplissage équipé d'une source de gaz chaud, i.e. environ 250°C, afin de régénérer l'adsorbant, c'est-à-dire évacuer/désorber le CO₂ piégé, et d'une source d'O₂ pressurisée pour remplir la cartouche avec de l'oxygène, à une pression n'excédant pas 10 bar par exemple.

Cependant, cette manière de procéder pour recycler les cartouches présente des inconvénients.

Ainsi, elle requiert la mise en place d'une logistique de transport complexe (i.e. récupération des cartouches utilisées, typiquement saturées en CO₂ et/ou vides, acheminement au centre, re-remplissage et réacheminement des cartouches pleines) et l'accès à un (des) centre de remplissage pas trop éloigné des sites d'utilisation des cartouches.

Ceci n'est pas toujours possible ou aisé.

Ainsi, sur une zone de combat ou un terrain d'opération militaire, par exemple un théâtre d'opération extérieur (OPEX), le re-remplissage de ce type de cartouches selon le processus décrit ci-dessus, est impossible du fait des contraintes de logistique existantes, des risques encourus, de l'absence ou inaccessibilité à un centre de remplissage... Or, la disponibilité d'oxygène peut être critique, en particulier en cas de blessure d'un soldat sur un théâtre d'opération nécessitant une administration immédiatement d'oxygène. Ce type de cartouche d'O₂ peut alors constituer une solution efficace pour délivrer un premier secours, dans l'attente d'une aide médicale plus adaptée.

Jeter simplement les cartouches après usage, c'est-à-dire sans les recycler, n'est pas idéal non plus car cela nécessite alors de multiplier le nombre de cartouches, ce qui entraine alors des contraintes logistiques et de stockage supplémentaires qui ne sont pas acceptables lorsqu'on se trouve dans un environnement où le poids et la taille des acheminements se doivent d'être limités, comme sur un terrain d'opération militaire ou une zone de combat, par exemple lors d'une OPEX ou analogue.

Au vu de cela, un problème est d'améliorer le conditionnement et recyclage des cartouches d'O₂, en particulier leur régénération et leur remplissage avec du gaz frais, y compris dans les zones ou sites éloignés ou risqués, tels les terrains d'opération militaires (e.g. OPEX), les zone de combat ou analogues.

Selon l'invention, il est proposé une installation de conditionnement d'une cartouche d'O₂ comprenant :
- une unité de production d'oxygène comprenant :
   ▪ une entrée d'admission d'air alimentée en air ambiant,
   ▪ au moins un module de séparation électrochimique configuré pour produire de l'oxygène ayant une pureté d'au moins 99%vol. à partir d'air ambiant provenant de l'entrée d'admission d'air, et rejetant un gaz-déchet,
   ▪ des premiers moyens de pilotage commandant ledit au moins un module de séparation électrochimique,
   ▪ une sortie d'oxygène pour fournir de l'oxygène produit par ledit au moins un module de séparation électrochimique, et
   ▪ une sortie de gaz-déchet délivrant le gaz-déchet provenant dudit au moins un module de séparation électrochimique, et
- une unité de remplissage comprenant :
   ▪ un dispositif de distribution de gaz relié fluidiquement à la sortie d'oxygène, à l'entrée d'admission d'air et à la sortie de gaz-déchet de l'unité de production d'oxygène, et par ailleurs à un conduit de jonction,
   ▪ des seconds moyens de pilotage pilotant le dispositif de distribution de gaz pour opérer une connexion fluidique entre le conduit de jonction et l'une desdites sortie d'oxygène, entrée d'admission d'air et sortie de gaz-déchet de l'unité de production d'oxygène,
   ▪ des moyens de réception de cartouche pour recevoir une cartouche d'O₂ munie de deux valves contrôlant les flux gazeux entrant et sortant de la cartouche d'O₂, le dispositif de distribution de gaz étant relié fluidiquement à la cartouche d'O₂ via le conduit de jonction lorsqu'une cartouche d'O₂ est positionnée dans les moyens de réception de cartouche, et
   ▪ un port de liaison à l'atmosphère en communication fluidique avec l'atmosphère ambiante.

Selon le mode de réalisation considéré, l'installation de conditionnement de l'invention peut comprendre l'une ou plusieurs des caractéristiques suivantes :
- le dispositif de distribution de gaz comprend une vanne rotative.
- le dispositif de distribution de gaz comprend une première entrée reliée fluidiquement à la sortie d'oxygène de l'unité de production d'oxygène.
- le dispositif de distribution de gaz comprend une deuxième entrée reliée fluidiquement à l'entrée d'admission d'air de l'unité de production d'oxygène.
- le dispositif de distribution de gaz comprend une troisième entrée reliée fluidiquement à la sortie de gaz-déchet.
- le dispositif de distribution de gaz comprend une quatrième entrée reliée fluidiquement au conduit de jonction.
- les moyens de réception de cartouche de l'unité de remplissage comprennent un logement à cartouche pour loger la cartouche d'O₂.
- les moyens de réception de cartouche de l'unité de remplissage comprennent des moyens de maintien de cartouche pour maintenir la cartouche d'O₂ dans le logement à cartouche.
- l'unité de remplissage comprend des moyens de déplacement coopérant avec les moyens de maintien de cartouche pour opérer un déplacement translatif de la cartouche d'O₂ dans le logement à cartouche.
- les moyens de déplacement comprennent au moins un actionneur mécanique, de préférence un moteur linéaire ou analogue.
- les moyens de déplacement sont contrôlés par les seconds moyens de pilotage.
- les moyens de maintien de cartouche comprennent un premier support et un second support agencés de part et d'autre du logement à cartouche, servant à maintenir mécaniquement la cartouche d'O₂ en position dans le logement à cartouche.
- les moyens de déplacement sont configurés pour agir sur les moyens de maintien de cartouche pour déplacer la cartouche d'O₂ et établir une connexion fluidique, au travers des valves de la cartouche d'O₂, entre le volume interne de la cartouche d'O₂, le conduit de jonction et le port de liaison à l'atmosphère relié à l'atmosphère ambiante, lorsqu'une cartouche d'O₂ munie de deux valves est logée dans le logement à cartouche.
- le premier support et/ou le second support sont mobile en translation, typiquement de haut en bas selon un axe vertical, lorsqu'ils est ou sont déplacés par les moyens de déplacement.
- ledit au moins un module de séparation électrochimique de l'unité de production d'oxygène comprend une ou plusieurs membranes céramiques.
- ledit au moins un module de séparation électrochimique comprend une ou plusieurs membranes céramiques dopées par un ou des électrolytes.
- le ou les électrolytes comprennent ou sont par exemple des composés à base de Cérium.
- l'unité de production d'oxygène comprend en outre des moyens d'aspiration de gaz, des moyens de chauffage de gaz et/ou des moyens d'échange thermique.
- la vanne rotative comprend un élément central formant un cylindre et un élément périphérique formant un couvercle, lesquels sont assemblés ou couplés l'un à l'autre.
- l'élément central formant un cylindre est traversé par un conduit coudé reliant un orifice latéral à un orifice central.
- l'élément périphérique formant couvercle comprend une paroi de fond en forme de disque et une paroi périphérique formant une bordure annulaire périphérique agencée autour de la paroi de fond et solidaire l'une de l'autre.
- la paroi de fond et la paroi périphérique délimitent un logement central configuré et dimensionné pour loger l'élément central après assemblage.
- l'élément périphérique comprend plusieurs évidements latéraux, i.e. tels des perçages, ouvertures, trous, orifices ou analogues, aménagés au travers de la bordure annulaire et en communication fluidique avec le logement central, de préférence un premier, un deuxième et un troisième évidement latéral.
- le conduit coudé est en communication fluidique avec le conduit de jonction, via la quatrième entrée.
- les premier, deuxième et troisième évidements latéraux de l'élément périphérique peuvent être mis en communication fluidique avec, respectivement, les première, deuxième et troisième entrées du dispositif de distribution de gaz.
- lorsque l'un des premier, deuxième et troisième évidements latéraux de l'élément périphérique est mis en communication fluidique avec l'une des première, deuxième et troisième entrées du dispositif de distribution de gaz, les deux autres évidements latéraux de l'élément périphérique sont obturés par la paroi de la bordure annulaire.
- la (ou chaque) membrane céramique est configurée pour permettre aux molécules d'O₂ contenues dans le gaz, i.e. de l'air, de la traverser tout en retenant les molécules d'autres espèces gazeuses, notamment celles d'azote, de CO₂, d'argon etc....
- l'unité de génération d'oxygène fournit de l'oxygène à une concentration d'au moins 99% en volume, de préférence d'au moins 99.5 % en volume, avantageusement entre 99.5% et 100%.
- les moyens de chauffage de gaz de l'unité de génération d'oxygène sont agencés entre les moyens d'aspiration de gaz et ledit au moins un module de séparation électrochimique.
- les moyens d'échange thermique de l'unité de génération d'oxygène sont agencés entre les moyens d'aspiration de gaz et les moyens de chauffage de gaz.
- le ou chaque module de séparation d'O₂ comprend une entrée d'air par lequel l'air véhiculé par la première ligne de gaz interne pénètre dans le ou chaque module de séparation d'O₂, avant sa séparation par la (ou les) membrane céramique agencée dans le ou chaque module de séparation d'O₂.
- les premiers et/ou seconds moyens de pilotage comprennent une ou des cartes de commande électroniques et une ou des unités de contrôle à microprocesseur(s), typiquement un (ou des) microcontrôleur.
- les premiers moyens de pilotage sont configurés pour commander le ou chaque module de séparation d'O₂.
- les premiers moyens de pilotage sont en outre configurés pour commander les moyens de chauffage et/ou les moyens d'aspiration des gaz.
- les premiers moyens de pilotage sont configurés pour alimenter électriquement la (ou les) membranes céramiques du ou de chaque module de séparation d'O₂.
- les premiers moyens de pilotage sont alimentés électriquement par des moyens alimentation électrique, tel le secteur (110/220 V) et/ ou une batterie rechargeable ou analogue.
- en phase de régénération (i.e. désorption), au moins une partie du CO₂ (et éventuellement de la vapeur d'eau) ayant été adsorbée par l'adsorbant de la cartouche est désorbée, i.e. libérée.
- l'installation comprend en outre des moyens alimentation électrique alimentant les composants de l'installation ayant besoin de courant électrique pour fonctionner, notamment les moyens de pilotage.

L'invention porte aussi sur un procédé de régénération et remplissage d'une cartouche d'O₂ munie de valves et contenant un matériau adsorbant, dans lequel on met en œuvre une installation selon l'invention, en particulier comme décrite ci-avant, pour :
a) positionner la cartouche d'O₂ dans les moyens de réception de cartouche,
b) maintenir la cartouche d'O₂ au moyen des moyens de maintien de cartouche,
c) opérer un déplacement des moyens de maintien de cartouche au moyen des moyens de déplacement, de préférence en translation, de manière à établir une connexion fluidique, au travers des valves de la cartouche d'O₂, entre le volume interne de la cartouche d'O₂, le conduit de jonction et le port de liaison à l'atmosphère relié à l'atmosphère ambiante,
d) régénérer le matériau adsorbant contenu dans le volume interne de la cartouche d'O₂ avec un gaz-déchet à une température d'au moins 300°C provenant de l'unité de production d'oxygène,
e) refroidir le matériau adsorbant contenu dans le volume interne de la cartouche d'O₂ avec de l'air provenant du port de liaison à l'atmosphère,
f) déplacement des moyens de maintien de cartouche par les moyens de déplacement, de préférence en translation, pour interrompre toute communication fluidique entre l'une des valves de la cartouche d'O₂ et le port de liaison à l'atmosphère,
g) remplir le volume interne de la cartouche d'O₂ avec de l'oxygène provenant de l'unité de production d'oxygène (3) jusqu'à obtenir une pression donnée, de préférence inférieure à 10 bar,
h) déplacement des moyens de maintien de cartouche par les moyens de déplacement, de préférence en translation, pour interrompre toute communication fluidique via l'autre des valves de la cartouche d'O₂, et
i) retirer la cartouche d'O₂ remplie avec de l'oxygène.

La cartouche d'O₂ récupérée qui a été régénérée et remplie avec de l'oxygène frais peut alors être réutilisée.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de conditionnement de cartouche d'O₂ selon la présente invention.
Fig. 2 schématise un mode de réalisation de l'unité de production d'oxygène de l'installation de conditionnement de Fig. 1.
Fig. 3 schématise un mode de réalisation de l'unité de remplissage de l'installation de conditionnement de Fig. 1.
Fig. 4 et Fig. 5 schématisent un mode de réalisation d'une vanne rotative utilisable dans l'unité de remplissage de Fig. 3.
Fig. 6 et Fig. 7 schématisent un mode de réalisation d'une cartouche d'O₂ 5 pouvant être régénérée et remplie grâce à l'installation de conditionnement de Fig. 1.
Fig. 8, Fig. 9 et Fig. 10 schématisent le fonctionnement de l'unité de remplissage de Fig. 3.

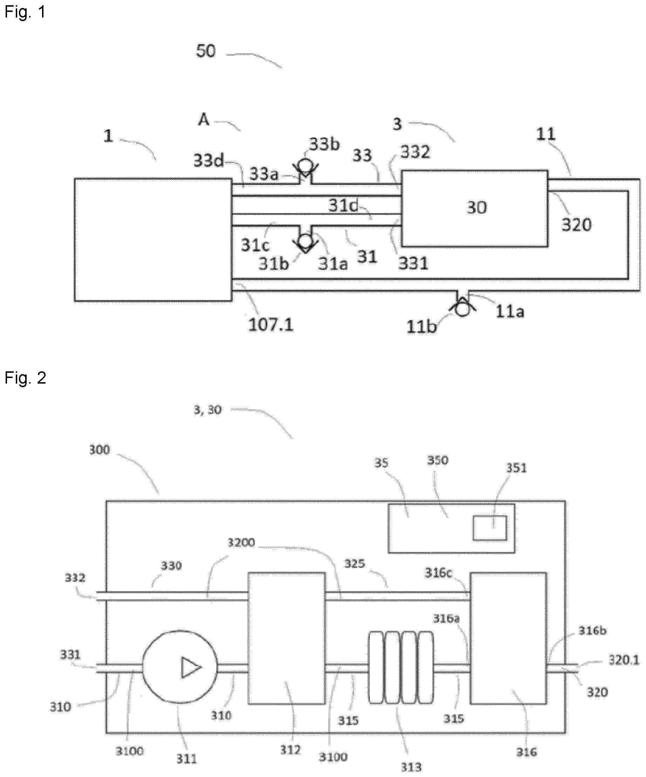

Fig. 1 schématise un mode de réalisation d'une station ou installation de conditionnement 50 d'un récipient de stockage de type cartouche d'O₂ 5 destiné à stocker de l'O₂ gazeux sous pression (e.g. pression <15 bar) selon la présente invention. Elle comprend une de production d'oxygène 3 et une unité de remplissage 1 coopérant l'une avec l'autre.

Cependant, bien que dans le mode de réalisation de Fig. 1, l'unité de production d'oxygène 3 et l'unité de remplissage 1 sont indépendantes, selon un autre mode de réalisation (non montré), ces unités pourraient être regroupées en un équipement unique intégrant lesdites unités de production d'oxygène 3 et de remplissage 1, par exemple au sein d'une même carcasse commune.

L'unité de production d'oxygène 3 faisant office de source d'oxygène 30 de haute pureté fournissant de l'oxygène ayant une pureté d'au moins >99% en vol. à une ligne de remplissage 11 alimentant une unité ou système de remplissage 1 servant à remplir une ou plusieurs cartouches d'O₂ utilisées, typiquement saturées en CO₂ et/ou vides, c'est-à-dire donc l'oxygène a été utilisé, typiquement une cartouche d'O₂ 5 à deux entrées opposées ayant été utilisée, comme celle décrite par EP4227575.

Comme illustré en Fig. 2, l'unité de production d'oxygène 3 comprend un ou des modules de séparation électrochimique 316 pour fournir l'oxygène, typiquement de l'oxygène pur ou quasiment pur (i.e. >99%vol.), par exemple ayant une pureté >99.95%vol., à une ligne d'acheminement de gaz, appelée ligne de remplissage 11, raccordée fluidiquement à une sortie d'oxygène 320.1 de l'unité de production d'oxygène 3.

Un exemple d'un module de séparation électrochimique 316 utilisable dans le cadre de la présente invention est décrit par le document : 49th International Conference on Environmental Systems; 7-11 July 2019; Boston (MA); ICES-2019-379; Solid State Electrochemical Oxygen Separation and Compression; M. Reisert et al.; p. 1-10*.*

De plus, l'unité de génération d'O₂ 3 comprend des premiers moyens de pilotage 35, i.e. un premier dispositif de pilotage, aussi appelé premier contrôleur, par exemple une première carte de commande électronique 350 et une première unité de contrôle 351 à microprocesseur(s), typiquement un (ou des) microcontrôleur. Les éléments électromécaniques de l'unité de génération d'O₂ 3, en particulier le ou les modules de séparation d'O₂ 316, ainsi que des moyens d'aspiration de gaz 311, sont alimentés électriquement et commandés par les premiers moyens de pilotage 35. Autrement dit, les premiers moyens de pilotage 35 pilotent, c'est-à-dire commandent le fonctionnement, du ou des modules de séparation d'O₂ 316 ainsi que les moyens d'aspiration de gaz 311.

L'unité de génération d'O₂ 3 comprend en outre, une première ligne de gaz interne 3100 comprenant une première portion amont appelée conduit d'admission 310 et une première portion aval appelée conduit d'entrée 315, et une seconde ligne de gaz interne 3200 comprenant une seconde portion amont appelée second conduit de sortie 325 et une seconde portion aval appelée conduit d'échappement 330.

Le conduit d'admission 310 interne comprend des moyens d'aspiration de gaz 311, i.e. un dispositif d'aspiration de gaz, tel qu'un dispositif ventilateur de type soufflante ou analogue, permettant d'aspirer du gaz, i.e. de l'air ambiant comme expliqué ci-après, pour le faire circuler dans le conduit d'admission 310 interne et ensuite au travers des moyens d'échange thermique 312, i.e. un (des) dispositif échangeur de chaleur, agencés aussi sur la première ligne de gaz interne 3100. Les moyens d'échange thermique 312 sont agencés entre le conduit d'admission 310 et le conduit d'entrée 315.

Après passage dans les moyens d'échange thermique 312, le flux d'air est récupéré et convoyé par le conduit d'entrée 315 de la première ligne de gaz interne 3100, lequel relie les moyens d'échange thermique 312 à des moyens de chauffage de gaz 313, i.e. un dispositif de chauffage de gaz, tel qu'un réchauffeur de gaz ou analogue.

Autrement dit, les moyens d'échange thermique 312 sont agencés, sur la première ligne de gaz interne 3100, entre les moyens d'aspiration de gaz 311 et les moyens de chauffage de gaz 313, qui sont eux-mêmes agencés sur la première ligne de gaz interne 3100. La première ligne de gaz interne 3100 amène le gaz, i.e. l'air, jusqu'au(x) module(s) de séparation d'O₂ électrochimique 316, lesquels sont agencés en aval des moyens de chauffage de gaz 313.

Les moyens de chauffage de gaz 313 sont configurés pour préchauffer les gaz véhiculés dans le conduit d'entrée 315 jusqu'à une température supérieure à 600°C avant qu'il ne soit amené à un (ou des) module de séparation d'O₂ électrochimique 316. Selon un autre mode de réalisation, on prévoit plusieurs modules de séparation d'O₂ électrochimique 316, par exemple agencés en série ou en parallèle.

Le ou chaque module de séparation d'O₂ 316 comprend une ou plusieurs membranes céramiques dopées par un (ou des) électrolyte(s) qui, sous l'action de la chaleur et d'un potentiel électrique appliqué par les premiers moyens de pilotage 35, typiquement par la première carte de commande 350, permet aux molécules d'O₂ contenues dans le gaz, i.e. de l'air, de traverser la ou les membranes céramiques et d'être récupérées dans un premier conduit de sortie 320 avec une sortie d'oxygène 320.1 qui alimente alors la ligne principale d'acheminement de gaz 320 en oxygène de très haute pureté, i.e. > 99%vol., préférentiellement > 99,50%vol., préférentiellement encore de l'ordre de 99,90%vol à 99.99%vol.

Une particularité de l'unité de génération d'O₂ 3 est que le potentiel électrique appliqué sur les membranes céramiques force les molécules d'O₂ à les traverser, indépendamment des conditions présentes dans le premier conduit de sortie 320, dans la limite des caractéristiques mécaniques de ces membranes céramiques. En d'autre termes, une pression positive (i.e. supérieure à la pression atmosphérique) dans le premier conduit de sortie 320 n'entrave pas le déplacement des molécules d'O₂ au travers des membranes céramiques. L'unité de génération d'O₂ 3 est capable de délivrer de l'O₂ pour des pressions dans le premier conduit de sortie 320 atteignant 10 bar relatifs ou plus. L'O₂ circule alors dans la ligne de remplissage 11, raccordée fluidiquement à la sortie d'oxygène 320.1 de l'unité de production d'oxygène 3.

La ligne de remplissage 11 présente par ailleurs un embranchement 11a, tel un conduit ou analogue, comprenant une valve d'échappement 11b qui s'ouvre à une pression déterminée, par exemple 10 bar relatifs, pour évacuer l'excès d'O₂ à l'atmosphère ambiante A, lorsque la pression dans la ligne de remplissage 11 devient supérieure à la pression d'échappement.

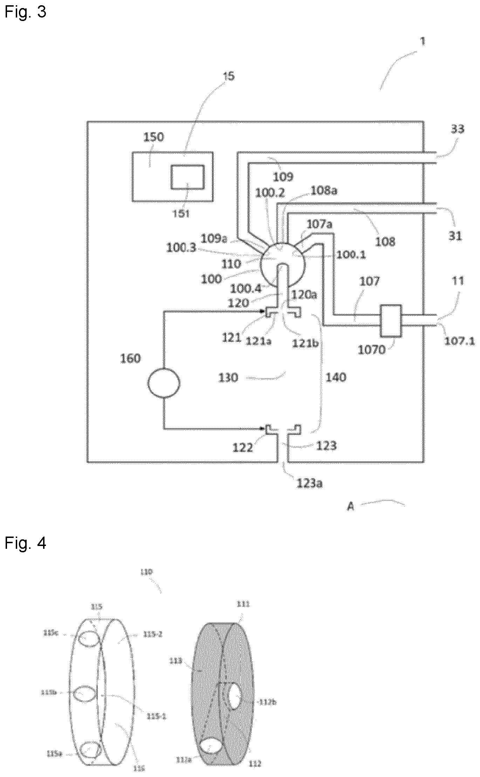

Lorsque l'unité de remplissage 1 est inactive, c'est-à-dire ne comportant pas une ou des cartouche(s) d'O₂ 5 comme illustré en Fig. 3, l'air est principalement capté à l'atmosphère ambiante A au travers de la valve d'admission 31b de l'embranchement 31a du conduit d'interconnexion 31. La valve d'admission 31b permet uniquement la circulation d'air provenant de l'atmosphère ambiante A vers l'intérieur du conduit d'interconnexion 31. L'air capté circule de l'embranchement 31a à la portion aval 31d du conduit d'interconnexion 31, donc en direction du conduit d'admission 310 de l'unité de génération d'O₂ 3, comme illustré en Fig. 1 à Fig. 3.

Autrement dit, le ou chaque module de séparation d'O₂ 316 de l'unité de génération d'O₂ 3 comprend :
- une entrée d'air 316a par lequel l'air véhiculé par la première ligne de gaz interne 3100 pénètre dans le ou chaque module de séparation d'O₂ 316, avant sa séparation par la (ou les) membrane céramique qui s'y trouve ;
- une première sortie 316b alimentant le premier conduit de sortie 320 en un flux d'oxygène de haute pureté produit par la (ou les) membrane céramique ; et
- une seconde sortie 316c alimentant la seconde ligne de gaz interne 3200 en gaz-déchet n'ayant pas traversé, c'est-à-dire ayant été retenu, par la (ou les) membrane céramique.

Ainsi, le flux de gaz obtenu circulant dans le conduit de sortie 320 est de l'oxygène de (très) haute pureté (>99 % vol.), par exemple d'au moins 99.95% vol. à 99.99% vol., à température ambiante, i.e. de l'ordre de 10 à 30°C, ne contenant pas ou des quantités négligeables d'autres composés. Autrement dit, la ou les membranes céramiques sont conçues pour ne laisser passer que les molécules d'oxygène.

Les autres composés présents, tel de l'oxygène (n'ayant pas traversé les membranes céramiques), l'azote, l'argon ou autres, ressortent du module de séparation d'O₂ 316 par la seconde sortie 316c et sont ensuite convoyés par la seconde ligne de gaz interne 3200 et rejetés à l'atmosphère en tant que gaz-déchet.

Plus précisément, du fait de la combinaison du gaz préchauffé et de l'effet Joule se manifestant dans le ou chaque module de séparation d'O₂ 316, le gaz-déchet contenant lesdits autres composés gazeux, qui est à une température de l'ordre de 850°C, ressort du module de séparation d'O₂ 316 par le second conduit de sortie 325 formant la portion amont de la seconde ligne de gaz interne 3200 qui convoie le gaz-déchet au travers les moyens d'échange thermique 312, i.e. un échangeur de chaleur, qui sont aussi agencé sur la seconde ligne de gaz interne 3200.

Dit autrement, les moyens d'échange thermique 312 sont traversés par la première ligne de gaz interne 3100 et la seconde ligne de gaz interne 3200, comme visible en Fig. 2.

Le flux de gaz-déchet en ressort alors par une sortie de gaz-déchet 332 du conduit d'échappement 330 formant la portion aval de la seconde ligne de gaz interne 3200, et est ensuite convoyé jusqu'à l'unité de remplissage de gaz 1, comme illustré en Fig. 1 et détaillé ci-après.

Les moyens d'échange thermique 312 sont configurés pour récupérer une partie de l'énergie calorifique, c'est-à-dire des calories, du gaz-déchet amené par le second conduit de sortie 325 de la seconde ligne de gaz interne 3200 et de la fournir au gaz circulant dans le conduit d'admission 310 de la première ligne de gaz interne 3100 pour le préchauffer et optimiser ainsi la consommation électrique de l'unité de génération d'O₂ 3.

Autrement dit, il s'opère un échange thermique, de préférence à contre-courant, entre le flux de gaz circulant dans le conduit d'admission 310, c'est-à-dire l'air, et le flux de gaz-déchet amené par le second conduit de sortie 325 au sein des moyens d'échange thermique 312. L'air y est donc réchauffé, alors que le gaz-déchet y est refroidi.

Après échange thermique au sein des moyens d'échange thermique 312, i.e. de l'échangeur de chaleur, le gaz-déchet évacué par le conduit d'échappement 330 à une température encore élevée, typiquement de l'ordre de 300°C environ, et contient moins de 21%vol. d'O₂ puisqu'une petite partie de l'oxygène présent dans le flux initial, a migré à travers le module de séparation d'O₂ 316 et a été évacué par le premier conduit de sortie 320.

Le débit d'O₂ fourni par l'unité de génération d'O₂ 3 est préférentiellement faible, par exemple de l'ordre de 1 L/min, de manière à limiter le poids et l'encombrement de l'unité de génération d'O₂ 3 mais aussi sa consommation électrique.

Tous les composants de l'unité de génération d'O₂ 3 sont agencés dans une carcasse ou un boitier 300 de protection rigide.

En fait, l'unité ou système de remplissage de gaz 1 est fluidiquement connecté à l'unité de production d'O₂ 3 au moyen de deux conduits parallèles, à savoir le premier conduit d'interconnexion 31 et un second conduit d'interconnexion 33 reliés fluidiquement, respectivement, au conduit d'admission 310 et au conduit d'échappement 330 de manière à pouvoir acheminer des flux d'air et de gaz-déchet entre ces unités 1, 3. De façon similaire, le second conduit d'interconnexion 33 comprenant un embranchement 33a, i.e. un conduit ou analogue, comprenant une valve d'échappement 33b qui s'ouvre à une pression prédéfinie, par exemple quelques dizaines de mb, pour évacuer l'excès de gaz-déchet à l'atmosphère ambiante A lorsque la pression dans le second conduit d'interconnexion 33 devient trop importante, c'est-à-dire supérieure à la pression d'échappement.

Fig. 3 schématise un mode de réalisation de l'unité de remplissage 1, faisant partie de l'installation 50 de conditionnement de cartouche d'O₂ 5, illustrée en Fig. 1, où est régénérée et remplie la cartouche d'O₂ 5 utilisée.

L'unité de remplissage 1 comprend des seconds moyens de pilotage 15, i.e. un second dispositif de pilotage, aussi appelé second contrôleur, typiquement une seconde carte de commande électronique 150 et une seconde unité de contrôle 151 à microprocesseur(s), typiquement un (des) microcontrôleur.

Tous les éléments électromécaniques du système ou unité de remplissage 1 sont alimentés électriquement et commandés par les seconds moyens de pilotage 15. Les seconds moyens de pilotage 15 sont eux-mêmes alimentés électriquement par une source de courant électrique (non montrée), par exemple une liaison au courant du secteur (110/220V) de type cordon électrique et prise de raccordement, ou une (ou des) batterie d'alimentation électrique, de préférence rechargeable, et/ou un transformateur de courant.

La seconde carte de commande 150, i.e. une carte électronique, intègre préférentiellement l'unité de contrôle 151 à microprocesseur et est configurée pour commander et par ailleurs analyser les signaux provenant des différents composants de l'unité de remplissage 1, tels que vannes, pompe, capteurs...

La seconde carte de commande 150 et les autres composants de l'unité de remplissage 1 sont agencés dans une carcasse ou boitier externe rigide, par exemple en polymère.

L'unité de remplissage 1 comprend un conduit de remplissage 107 avec un orifice d'entrée 107.1 qui est relié fluidiquement à la ligne de remplissage 11. La ligne de remplissage 11 vient se raccorder fluidiquement, par son extrémité aval, au conduit de remplissage 107 via un système de raccordement comprenant des connecteurs réciproques, par exemple de type mâle/femelle, permettant d'assurer une connexion mécanique et fluidique, et par ailleurs au premier conduit de sortie 320 de l'unité de génération d'O₂ 3. Autrement dit, l'entrée de l'oxygène produit par l'unité de génération d'O₂ 3 se fait dans la portion amont ou portion d'entrée du conduit de remplissage 107 du système de remplissage 1, dans lequel est agencé par ailleurs un capteur de pression 1070.

En aval du capteur de pression 1070, le conduit de remplissage 107 débouche en son extrémité 107a sur un dispositif de distribution de gaz 100, qui est ici une vanne rotative 110.

Le dispositif de distribution de gaz 100 comprend :
- une première entrée 100.1 reliée fluidiquement à la sortie d'oxygène 320 de l'unité de production d'oxygène 3, via le conduit de remplissage 107 et la ligne de remplissage 11,
- une deuxième entrée 100.2 reliée fluidiquement à l'entrée d'admission d'air 331 de l'unité de production d'oxygène 3, via le conduit de refroidissement 108 et un premier conduit d'interconnexion 31,
- une troisième entrée 100.3 reliée fluidiquement à la sortie de gaz-déchet 332, via le conduit de régénération 109 et le second conduit d'interconnexion 33, et
- une quatrième entrée 100.4 reliée fluidiquement à un conduit de jonction 120.

Le conduit de refroidissement 108 est relié au premier conduit d'interconnexion 31, lui-même connecté au conduit d'admission 310 de l'unité de génération d'O₂ 3. A son extrémité 108a, le conduit de refroidissement 108 communique avec la deuxième entrée 100.2 du dispositif de distribution de gaz 100, à savoir ici la vanne rotative 110.

De façon similaire, le conduit de régénération 109 est relié au second conduit d'interconnexion 33, lui-même connecté à la sortie de gaz-déchet 332 du conduit d'échappement 330 de l'unité de génération d'O₂ 3. A son extrémité 109a, le conduit de régénération 109 communique avec la troisième entrée 100.3 du dispositif de distribution de gaz 100, à savoir ici la vanne rotative 110.

Fig. 4 est une vue schématique éclatée d'un mode de réalisation du dispositif de distribution de gaz 100 qui est ici une vanne rotative 110 comprenant deux éléments principaux assemblés l'un à l'autre, à savoir :
- un élément central 111 formant un cylindre plein traversé par un conduit coudé 112 reliant un orifice latéral 112a à un orifice central 112b ; et
- un élément périphérique 115 formant un « couvercle ». Il comprend une paroi de fond 115-1 en forme de disque et une paroi périphérique 115-2 formant une bordure annulaire périphérique autour de la paroi de fond. La paroi de fond 115-1 et la paroi périphérique 115-2 délimitent un espace ou logement central 116 configuré et dimensionné pour loger l'élément central 111, i.e. cylindre plein, après assemblage. L'élément périphérique 115 présente trois évidements latéraux 115a-115c aménagé dans la bordure annulaire et en communication fluidique avec le logement central 116.

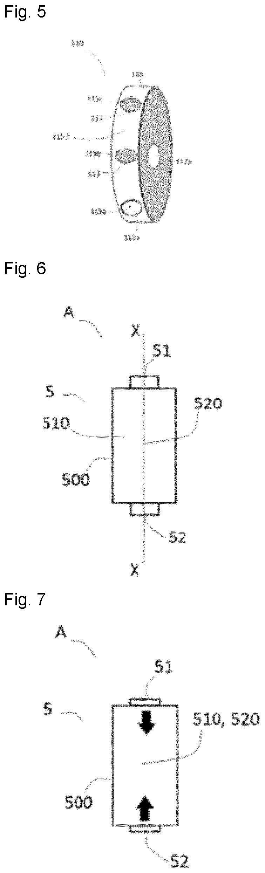

Fig. 5 illustre l'assemblage de l'élément central 111 et élément périphérique 115 formant « couvercle » de Fig. 4 pour former ainsi la vanne rotative 110.

Dans cette configuration, l'orifice latéral 112a du conduit 112 du cylindre plein formant l'élément central 111 est en vis-à-vis de l'orifice latéral 115a de l'élément périphérique 115 formant couvercle. A contrario, les orifices latéraux 115b, 115c de l'élément périphérique 115 sont occultés par la paroi latérale 113, i.e. la paroi périphérique 115-2, de l'élément central, i.e. cylindre plein. L'orifice latéral 115a est donc en communication fluidique avec l'orifice central 112b du conduit 112, tandis que les orifices latéraux 115b, 115c de l'élément périphérique 115 sont « bouchés » par la paroi latérale 113 de l'élément central 111.

L'absence de fuites entre l'élément périphérique 115, l'élément central 111 et leurs orifices respectifs, peut être assurée en intégrant des joints (non représentés), par exemple des joints toriques, autour des orifices latéraux 115a-115c de l'élément périphérique 115.

En outre, les orifices 115a-115c de la vanne rotative 110 sont respectivement raccordés, par exemple emmanchés à force, soudés ou autre, au conduit de régénération 109, au conduit de refroidissement 108 et au conduit de remplissage 107.

Par ailleurs, l'orifice central 112b du conduit coudé 112 de l'élément central 111 est lui-même raccordé au conduit de jonction 120, comme illustré en Fig. 3. Cette liaison est de type circulaire en ce sens que l'élément central 111 ayant une forme de cylindre plein, peut effectuer un mouvement de rotation autour de l'axe défini par l'orifice central 112b, physiquement raccordé au conduit de jonction 120. Un tel mouvement de rotation peut être obtenu par le couplage mécanique à un actionneur rotatif (non montré), par exemple un moteur pas-à-pas contrôlé par la seconde carte de commande 150, permettant alors à l'élément central 111 d'effectuer un mouvement de rotation dans l'élément périphérique 115.

Moyennant un contrôle approprié par les seconds moyens de pilotage 15, typiquement par la seconde carte de commande 150, on peut configurer le dispositif de distribution de gaz 100, à savoir ici la vanne rotative 110, de manière à sélectionner une configuration fluidique, c'est-à-dire, sur la Fig. 5, de connecter fluidiquement le conduit de régénération 109 au conduit de jonction 120, alors que le conduit de refroidissement 108 et le conduit de remplissage 107 sont obturés et ne peuvent pas véhiculer de gaz.

Autrement dit, le dispositif de distribution de gaz 100, c'est-à-dire ici la vanne rotative 110, est piloté par la seconde carte de commande 150 de sorte de réaliser une communication fluidique entre le conduit de régénération 109 et le conduit de jonction 120, et donc l'intégralité du gaz circulant dans le conduit de régénération 109 est acheminé dans le conduit de jonction 120.

Une telle configuration peut être modifiée par les seconds moyens de pilotage 15, typiquement la seconde carte de commande 150, de manière à connecter fluidiquement le conduit de refroidissement 108 au conduit de jonction 120, ou le conduit de remplissage 107 au même conduit de jonction 120.

En d'autres termes, en fonction de la configuration du dispositif de distribution de gaz 100, à savoir ici de la vanne rotative 110, piloté par les seconds moyens de pilotage 15, le conduit de jonction 120 est fluidiquement connecté, soit au conduit de régénération 109, soit au conduit de refroidissement 108, soit au conduit de remplissage 107. Préférentiellement, le conduit de jonction 120 est flexible et peut s'étendre et se rétracter selon les besoins.

Le système ou unité de remplissage 1 comprend par ailleurs des moyens de réception de cartouche 140 permettant de recevoir et maintenir une cartouche d'O₂ 5 utilisée, typiquement saturée en CO₂ et/ou vide, pendant son processus de remplissage avec de l'oxygène provenant de l'unité de production d'oxygène 3.

Les moyens de réception de cartouche 140 comprennent un logement à cartouche 130 pour recevoir, i.e. loger, la cartouche d'O₂ 5 utilisée, c'est-à-dire saturée en CO₂ et/ou vide (i.e. dont la (quasi)totalité de l'oxygène a été utilisé) devant être régénérée et remplit de gaz frais, et des moyens de maintien de cartouche 121, 122, à savoir, dans le mode de réalisation de Fig. 3, un premier support 121 et un second support 122 agencés de part et d'autre du logement à cartouche 130, servant à maintenir mécaniquement la cartouche d'O₂ 5 en position dans le logement à cartouche 130 et à assurer une communication fluidique, lorsque nécessaire, entre le volume interne de la cartouche d'O₂ 5 et l'un et/ou l'autre du conduit de jonction 120 et le port (i.e. orifice de décharge) de liaison à l'atmosphère 123a, comme expliqué ci-après.

A l'extrémité 120a du conduit de jonction 120 est agencé le premier support 121. Il est de forme circulaire et présente un premier fond plat 121a au centre duquel est agence un premier orifice 121b en relation fluidique avec le conduit de jonction 120.

De préférence, le premier support 120 est mobile en translation de bas en haut sur Fig. 3, c'est-à-dire qu'il peut être déplacé selon des mouvements de translation verticaux en réponse à une action de la seconde carte de commande 150.

Pour ce faire, le premier support 121 est préférentiellement couplé mécaniquement à des moyens (ou un dispositif) de déplacement 160, i.e. comprenant un actionneur mécanique ou analogue, tel un moteur linéaire ou analogue, assurant le mouvement de translation du premier support 121 en réponse au pilotage, i.e. commande, de la seconde carte de commande 150 des seconds moyens de pilotage 15.

Autrement dit, les moyens de déplacement 160 coopèrent avec les moyens de maintien de cartouche 121, 122 pour déplacer la cartouche d'O₂ 5 en translation verticale, c'est-à-dire selon son axe longitudinal, lorsqu'elle est positionnée dans le logement à cartouche 130.

En regard du premier support 121, qui est séparé par le logement à cartouche 130, c'est-à-dire un espacement ou compartiment, conçu et dimensionné pour recevoir une cartouche d'O₂ 5 à régénérer et à remplir, se trouve un second support 122, qui est préférentiellement identique au premier support 121, c'est-à-dire présentant lui-aussi un second fond plat 122a au centre duquel se trouve un second orifice 122b en relation fluidique avec un conduit de liaison à l'atmosphère 123 qui est relié fluidiquement avec l'atmosphère ambiante A, via un port ou orifice de liaison à l'atmosphère 123a.

Préférentiellement, le conduit de liaison à l'atmosphère 123 est de type flexible et peut s'étendre et se rétracter.

Le second support 122 est, tout comme le premier support 121, mobile en translation, c'est-à-dire de haut en bas, lorsqu'il est déplacé par les moyens de déplacement 160, tel un actionneur mécanique, par exemple de type moteur linéaire ou analogue, lorsqu'ils sont commandés par les seconds moyens de pilotage 15, en particulier la seconde carte de commande 150.

Un mode de réalisation d'un récipient ou cartouche d'O₂ 5 à deux valves 51, 52 pouvant être régénérée et remplie par l'installation de conditionnement 1 selon l'invention est schématisé en Fig. 6 et Fig. 7.

Ce type de cartouche d'O₂ 5 est bien décrit par EP4227575 auquel on peut se référer pour mieux comprendre son fonctionnement, notamment son utilisation pour fournir de l'oxygène à un utilisateur, typiquement un patient ou une personne ayant besoin d'inhaler de l'oxygène.

Plus précisément, une telle cartouche d'O₂ 5 comprend un corps 500 ou réceptacle allongé, par exemple en un alliage d'aluminium ou un autre métal, définissant un volume interne 510 recevant l'oxygène et contenant (au moins) un matériau adsorbant 520 sélectif pour le CO₂. Le corps 500 est ici de forme cylindrique et contient le matériau adsorbant 520.

Aux extrémités du corps 500 cylindrique sont agencées les première et seconde valves 51, 52, de préférence identiques, contrôlant les entrées et sorties de gaz de la cartouche d'O₂ 5, notamment lors du processus de remplissage, comme expliqué ci-après.

En position de repos (cf. Fig. 6), les première et seconde valves 51, 5 sont fermées de manière à isoler le volume interne 510 de la cartouche d'O₂ 5 de l'atmosphère ambiante A et d'empêcher les entrées et sorties d'oxygène dudit volume interne 510. Dès lors, lorsqu'elle n'est pas utilisée ou sollicitée, la cartouche d'O₂ 5 peut contenir et stocker de l'oxygène pressurisé, de préférence à une pression inférieure ou égale à 10 bar relatif.

A l'inverse, en position active, typiquement suite à une action mécanique sur celles-ci, les première et seconde valves 51, 52 peuvent « rentrer » dans le corps 500, c'est-à-dire se déplacer en réalisant un mouvement de translation vertical en direction du centre du corps 500, comme illustre en Fig. 7. Les première et seconde valves 51, 52 établissent alors une connexion fluidique entre le volume de gaz 510 contenu dans le corps 500 et l'atmosphère ambiante A, i.e. l'air ambiant.

Bien entendu, l'une des première et seconde valves 51, 52 peut être en position active, alors que l'autre est en position de repos, et inversement.

Lorsqu'elle est utilisée pour fournir de l'oxygène à un utilisateur et capter le CO₂ contenus dans les gaz expirés par l'utilisateur, les valves 51, 52 sont rentrées comme illustré en Fig. 7 et expliqué par EP4227575, suite au raccordement auxdites valves 51, 52, d'une part, d'un sac-réservoir servant à recueillir de l'oxygène et, d'autre part, d'une interface respiratoire, tel un masque respiratoire facial. Lors des échanges gazeux se produisant entre la cartouche 5 et les poumons de l'utilisateur, l'adsorbant 520 va progressivement piéger le CO₂ expiré qui va alors être retenu par ledit adsorbant 520 jusqu'à sa saturation par des espèces CO₂.

Arrivé à saturation, l'adsorbant 520 va devoir subit une régénération afin d'en éliminer les espèces CO₂ et l'oxygène consommé remplacé par de l'oxygène 'frais'. La régénération suivie d'un remplissage de la cartouche d'O₂ 5 avec de l'oxygène 'frais' s'effectue avec une installation de conditionnement selon l'invention.

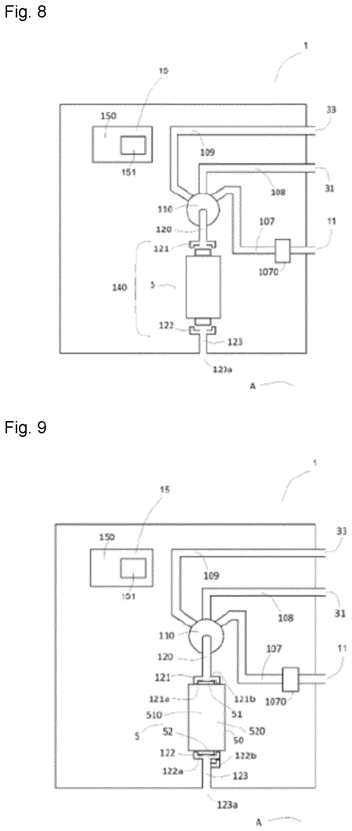

Fig. 8 à Fig. 10 illustrent une séquence de régénération par le système ou unité de remplissage 1 de l'invention, comme illustré sur Fig.1 à Fig.3, d'une cartouche d'O₂ 5 utilisée, c'est-à-dire dont l'adsorbant 520 est saturée en CO₂ et dont une partie au moins de l'oxygène qu'elle contenait a été utilisée, i.e. inhalé par un utilisateur, tel un patient.

Fig. 8 schématise une mise en place de la cartouche d'O₂ 5 dans le système ou unité de remplissage 1 de l'invention, dans les moyens de réception de cartouche 140, c'est-à-dire ici entre les premier et second supports 121, 122, via par exemple une (ou des) ouverture pratiquée dans la carcasse du système 1, de préférence dotée d'une ou des portes ou panneaux glissants (non représentés).

Le maintien en position de la cartouche d'O₂ 5 dans les moyens de réception de cartouche 140 peut être réalisé par tout moyen ou dispositif adapté, par exemple par clipsage, par des moyens de serrage, par moyens à vis, ... ou autres.

Sur Fig. 8, on voit que les seconds moyens de pilotage 15, en particulier la seconde carte de commande 150, commandent le dispositif de distribution de gaz 100, i.e. la vanne rotative 110, pour opérer une communication fluidique entre le conduit de régénération 109 et le conduit de jonction 120. La cartouche d'O₂ 5 à régénérer est placée par l'utilisateur entre les premier et second supports 121, 122.

Ensuite, comme montré en Fig. 9, les seconds moyens de pilotage 15, en particulier la seconde carte de commande 150, pilotent les premier et second supports 121, 122 pour qu'ils se déplacent au contact des première et seconde valves 51, 52 et viennent appuyer dessus pour ouvrir les valves 51, 52. Le premier support 121 opère donc un mouvement de translation vers le bas et le premier fond plat 121a du premier support 121 repousse alors la valve 51 vers le bas, alors que, dans le même temps, le second support 122 opère un mouvement de translation vers le haut et le second fond plat 122a du second support 122 repousse la valve 52 vers le haut. Il s'établit alors une continuité fluidique entre le volume de gaz 510 de la cartouche d'O₂ 5 et les premier et second orifices 121b, 122b des premier et second supports 121, 122, donc aussi entre le conduit de jonction 120 et le conduit de liaison à l'atmosphère 123.

Du fait de la configuration de la vanne rotative 110, i.e. du dispositif de distribution de gaz 100, le conduit de régénération 109 et le conduit de liaison à l'atmosphère 123 sont également mis en connexion fluidique, donc communiquent tous les deux avec l'atmosphère ambiante A, par l'intermédiaire du port de liaison à l'atmosphère 123a.

Dans le cadre de l'invention, on utilise l'unité de génération d'O₂ 3 pour opérer la régénération du matériau adsorbant 520 contenu dans le volume interne 510 de la cartouche 5, lequel est chargé de CO₂, voire même saturé en CO₂. En effet, comme illustré sur Fig. 2, le conduit d'échappement 330 de l'unité de génération d'O₂ 3 convoie un gaz appauvri en O₂ (i.e. de concentration inférieure à 21% vol. d'O₂) à haute température, c'est-à-dire à au moins 300°C environ. Or, ce conduit d'échappement 330 est fluidiquement relié au second conduit d'interconnexion 33, lui-même fluidiquement relié au conduit de régénération 109 du système ou unité de remplissage 1.

Dès lors, le gaz circulant dans le conduit de régénération 109 est un gaz à haute température (e.g. >300°C) qui est dirigé ensuite dans le conduit de jonction 120 et la cartouche d'O₂ 5, du fait de la configuration pneumatique de la vanne rotative 110. Ce gaz va pénétrer la cartouche d'O₂ 5, via la première vanne 51, entraîner une partie du CO₂ et de la vapeur d'eau contenus dans la première cartouche d'O₂ 5 et en ressortir par la seconde vanne 52.

En d'autres termes, le débit d'air sortant de la cartouche d'O₂ 5 via la vanne 52 est enrichi en CO₂ et en vapeur d'eau. Bien que le gaz circulant dans le conduit de régénération 109 contienne des traces (i.e. très faibles teneurs) de vapeur d'eau et de CO₂, puisqu'il émane de l'air ambiant ayant circulé dans le conduit d'admission 310 de l'unité de génération d'O₂ 3, la température élevée, i.e. d'au moins 300°C environ, permet d'assurer une désorption complète de la cartouche d'O₂ 5 sans entrainer par ailleurs d'adsorption temporaire des traces de vapeur d'eau et de CO₂ susceptibles de s'y trouver.

Le débit gazeux sortant de la cartouche d'O₂ 5, via la seconde vanne 52, circule dans le conduit de liaison à l'atmosphère 123, avant d'être évacué à l'air ambiant, via le port de liaison à l'atmosphère 123a du conduit de liaison à l'atmosphère 123. Autrement dit, on obtient une régénération de la cartouche d'O₂ 5 par le flux de gaz chauffé provenant de l'unité de génération d'O₂ 3.

Le gaz circulant dans le conduit de régénération 109 n'est qu'une partie du gaz issu du conduit d'échappement 330 de l'unité de génération d'O₂ 3. En effet, une partie de celui-ci s'échappe, via la valve d'échappement 33b du second circuit d'interconnexion 33, alors que le complément circule dans la portion avale 33d du second circuit d'interconnexion 33 et ensuite dans le conduit de régénération 109.

Par ailleurs, du fait de la configuration de la vanne rotative 110, la première entrée 100.1, reliée fluidiquement au conduit de remplissage 107 et à la sortie d'oxygène 320 de l'unité de production d'oxygène 3 est obturée, alors que la deuxième entrée 100.2 reliée fluidiquement au conduit de refroidissement 108 et à l'entrée d'admission d'air 331 de l'unité de production d'oxygène 3 est bouchée également.

Par conséquent, l'oxygène produit par l'unité de production d'oxygène 3 ne peut s'écouler librement dans la ligne de remplissage 11 et la pression dans cette dernière va augmenter progressivement jusqu'à atteindre une valeur supérieure à la pression d'échappement de la valve d'échappement 11b, pour se décharger alors à l'atmosphère ambiante A.

Comme illustré en Fig. 2, l'unité de génération d'O₂ 3 comprend un conduit d'admission 310 avec une entrée (i.e. orifice) d'admission d'air 331 alimenté en air ambiant provenant du système ou unité de remplissage 1, via le premier conduit d'interconnexion 31.

Un ventilateur 311 de type soufflante agencé dans le conduit d'admission 310, en aval de l'orifice d'admission 331, permet d'aspirer l'air ambiant acheminé par le premier conduit d'interconnexion 31.
Le conduit d'admission 310 de l'unité de génération d'O₂ 3 de Fig. 2 est relié au conduit de refroidissement 108 du système ou unité de remplissage 1 de Fig. 3, via le premier conduit d'interconnexion 31. De la même manière, la deuxième entrée 100.2 reliée fluidiquement au conduit de refroidissement 108 étant obturée, l'air capté circule alors par la valve d'admission 31b, l'embranchement 31a jusqu'à la portion avale 31d du conduit d'interconnexion 31 et au conduit d'admission 310 de l'unité de génération d'O₂ 3.

Une fois la cartouche d'O2 5 régénérée, il convient de la refroidir par balayage gazeux. La seconde carte de commande 150 pilote alors le dispositif de distribution de gaz 100, préférentiellement ici une vanne rotative 110, afin de réaliser une communication fluidique entre le conduit de refroidissement 108 et le conduit de jonction 120, via les deuxième et quatrième entrées 100.2, 100.4. Il s'établit alors une communication fluidique entre le conduit de refroidissement 108 et le conduit de liaison à l'atmosphère 123, au travers de la cartouche 5 lorsque celle-ci est agencée dans les moyens de réception de cartouche 140.

Etant donné que le conduit de liaison à l'atmosphère 123 est relié à l'air ambiant via le port de liaison à l'atmosphère 123a, le ventilateur 311 agencé dans le conduit d'admission 310 de l'unité de génération d'O₂ 3 peut aspirer de l'air ambiant au travers du port de liaison à l'atmosphère 123a et le complémenter au travers de la valve d'admission 31b. Le débit circulant dans la portion aval 31d du premier circuit d'interconnexion 31, relié au conduit d'admission 310 de l'unité de génération d'O₂ 3 est alors la somme du débit circulant au travers de la cartouche 5 et de la valve d'admission 31b. Le dimensionnement de la valve d'admission 31b peut être ajusté pour que le débit circulant dans ladite cartouche 5 soit suffisant à son refroidissement.

Une partie du gaz aspiré (i.e. de l'air) transite alors dans, respectivement, le conduit de liaison à l'atmosphère 123, le volume interne 510 de la cartouche d'O₂ 5 et le conduit de refroidissement 108 du système de remplissage 1, pour ensuite en sortir via le premier conduit d'interconnexion 31, réalisant une connexion fluidique avec le conduit d'admission 310.

Autrement dit, l'unité de génération d'O₂ 3 est alimentée en air par le conduit d'interconnexion 31 (et la valve d'admission 31b) qui vient se raccorder fluidiquement au conduit d'admission 310 par une de ses extrémités, ledit conduit d'interconnexion 31 étant raccordé fluidiquement, via son autre extrémité, au conduit de refroidissement 108 lui-même relié au conduit de liaison à l'atmosphère 123 en communication avec l'atmosphère, via l'orifice ou port de liaison à l'atmosphère 123a.

Le gaz aspiré à température ambiante (par ex. 20 à 25°C) va refroidir le matériau adsorbant 520 de la cartouche d'O₂ 5 en la traversant, alors que celle-ci a été précédemment chauffée par les gaz chauds (>300°C) ayant transité par le conduit de régénération 109 et le conduit de liaison à l'atmosphère 123.

Le matériau adsorbant 520 contenu dans la cartouche d'O₂ 5 se retrouve alors débarrassé de CO₂ et de vapeur d'eau résiduels du fait de la régénération, et à température ambiante du fait du balayage opéré avec le flux d'air atmosphérique.

Du fait de la configuration de la vanne rotative 110, la première entrée 100.1, reliée fluidiquement au conduit de remplissage 107 et à la sortie d'oxygène 320 de l'unité de production d'oxygène 3 est obturée et l'oxygène produit s'échappe alors par la valve d'échappement 11b comme décrit ci-dessus. De façon analogue, la troisième entrée 100.3 reliée fluidiquement à la sortie de gaz-déchet 332, via le conduit de régénération 109 et le second conduit d'interconnexion 33 est obturée. Par conséquent, le gaz-déchet produit par l'unité de production d'oxygène 3 ne peut s'écouler librement dans second conduit d'interconnexion 33 et la pression dans ce dernier va augmenter progressivement jusqu'à atteindre une valeur supérieure à la pression d'échappement de la valve d'échappement 33b, pour se décharger alors à l'atmosphère ambiante A.

Les étapes suivantes portent sur le re-remplissage de la cartouche d'O₂ 5 de sorte qu'elle puisse être réutilisée, i.e. recyclée.

Pour ce faire, la seconde carte de commande 150 pilote le dispositif de distribution de gaz 100, i.e. la vanne rotative 110, afin de réaliser une communication fluidique entre le conduit de remplissage 107 et le conduit de jonction 120. Il existe alors une relation fluidique entre le conduit de remplissage 107, le conduit de jonction 120, la cartouche d'O₂ 5, le conduit de liaison à l'atmosphère 123 et par extension l'air ambiant A. Lors de cette phase, la pression initiale dans respectivement la ligne de remplissage 11 et le conduit de remplissage 107, est égale à la pression d'échappement de la valve d'échappement 11b, mais s'abaissera brusquement à la pression atmosphérique, ce qui aura pour conséquence de mettre la valve d'échappement 11b en position fermée.

Comme illustré en Fig. 2, l'unité de génération d'O₂ 3 comprend un premier conduit de sortie 320, délivrant de l'O₂, relié à la ligne de remplissage 11, laquelle est reliée au conduit de remplissage 107 du système de remplissage 1.

L'apport d'oxygène de l'unité de génération d'O₂ 3 qui transite dans la ligne de remplissage 11, le conduit de remplissage 107 et ainsi le conduit de jonction 120 traverse la cartouche d'O₂ 5 pour en sortir via la vanne 52 et le conduit de liaison à l'atmosphère 123 jusqu'à atteindre l'air ambiant A. Ceci permet de chasser l'air précédemment présent comme volume de gaz 54 dans le réceptacle 50 et de le remplacer par de l'oxygène.

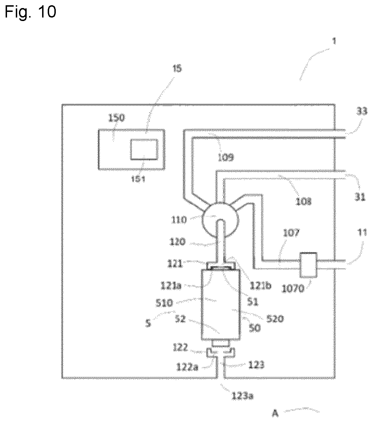

Une fois la cartouche d'O₂ 5 remplie d'O₂ à pression ambiante, il convient alors de la pressuriser. Cette étape de pressurisation est schématisée en Fig. 10.

Plus précisément, pour ce faire, la seconde carte de commande 150 pilote le second support 122 afin de lui ordonner un mouvement de translation vers le bas. Le second fond plat 122a du second support 122 libère la seconde vanne 52 de la cartouche d'O₂ 5 qui revient alors en position de repos. Dans le même temps, le premier support 121 garde sa position et contraint la première vanne 51 de la cartouche d'O₂ 5 à rester dans une position où il existe une relation fluidique entre le conduit de jonction 120 et le volume de gaz 54 de la cartouche d'O₂ 5. En d'autre termes, la seconde vanne 52 isole le volume de gaz 54 de la cartouche d'O₂ du second conduit 122, alors que le conduit de jonction 120 est toujours en relation fluidique avec le volume de gaz 54 de la cartouche d'O₂.

Comme illustré en Fig. 2, dans le mode de réalisation proposé, l'unité de génération d'O₂ 3 est capable de produire de l'O₂ jusqu'à une pression pouvant atteindre 10 bar, mesurée dans le premier conduit de sortie 320.

Ainsi, comme illustré en Fig. 10, le système de remplissage 1 oriente l'O₂ produit par l'unité de génération d'O₂ 3, via le premier conduit de sortie 320, vers respectivement la ligne de remplissage 11 et le conduit de remplissage 107.

L'oxygène produit par l'unité de génération d'O₂ 3 est alors dirigé dans la cartouche d'O₂ 5, c'est-à-dire que la cartouche 5 se remplit d'O₂.
Pendant que la cartouche d'O₂ 5 reçoit de l'O₂, la seconde carte de commande 150 monitore la pression retournée par le capteur de pression 1070, agencé dans le conduit de remplissage 1070. Lorsque la carte de commande détermine que la pression dans la cartouche d'O2 5 a atteint 10 bar, elle pilote le premier support 121 de façon à libérer la première vanne 51 et revenir à la configuration illustrée en Fig. 8.

La deuxième entrée 100.2 reliée fluidiquement au conduit de refroidissement 108 étant obturée, l'air capté circule alors par la valve d'admission 31b, l'embranchement 31a jusqu'à la portion avale 31d du conduit d'interconnexion 31 et au conduit d'admission 310 de l'unité de génération d'O₂ 3. La troisième entrée 100.3 reliée fluidiquement à la sortie de gaz-déchet 332, via le conduit de régénération 109 et le second conduit d'interconnexion 33 étant bouchée, le gaz-déchet produit par l'unité de production d'oxygène 3 ne peut s'écouler librement dans second conduit d'interconnexion 33 et la pression dans ce dernier va augmenter progressivement jusqu'à atteindre une valeur supérieure à la pression d'échappement de la valve d'échappement 33b, pour se décharger alors à l'atmosphère ambiante A.

Le dispositif de distribution de gaz 100 de type vanne rotative 110 et des valves d'admission et d'échappement 11b, 31b, 33b servent à diriger les différents flux de gaz. Alternativement, d'autres moyens, telles des valves tout ou rien, proportionnelles à simple ou multiple ports, peuvent être employés aux mêmes fins.

A l'issue de ces différentes phases, La cartouche d'O₂ 5 est alors pleine d'oxygène et peut être retirée en vue d'un futur usage, c'est-à-dire qu'elle se retrouve alors régénérée et prête à l'emploi.

Le système de remplissage 1 permet alors de permettre de recycler des cartouches d'O₂ 5 ayant été utilisées, donc d'éviter leur mise au rebut.

Il présente en outre l'avantage de pouvoir être utilisé sur site et ce quel que soit le site d'utilisation, c'est-à-dire y compris dans les zones compliquées, donc ne nécessite pas de centre de remplissage à proximité, ni de logistique compliquée pour récupérer les cartouches d'oxygène utilisées, typiquement saturées en CO₂ et/ou vides, puis ramener les cartouches 5 à nouveau remplies sur leur site d'utilisation.

## Revendications

1. Installation (50) de conditionnement d'une cartouche d'O₂ (5) comprenant :
- une unité de production d'oxygène (3) comprenant :
▪ une entrée d'admission d'air (331) alimentée en air ambiant,
▪ au moins un module de séparation électrochimique (316) configuré pour produire de l'oxygène ayant une pureté d'au moins 99%vol. à partir d'air ambiant provenant de l'entrée d'admission d'air (331), et rejetant un gaz-déchet,
▪ des premiers moyens de pilotage (35) commandant ledit au moins un module de séparation électrochimique (316),
▪ une sortie d'oxygène (320) pour fournir de l'oxygène produit par ledit au moins un module de séparation électrochimique (316), et
▪ une sortie de gaz-déchet (332) délivrant le gaz-déchet provenant dudit au moins un module de séparation électrochimique (316), et
- une unité de remplissage (1) comprenant :
▪ un dispositif de distribution de gaz (100) relié fluidiquement à la sortie d'oxygène (320), à l'entrée d'admission d'air (331) et à la sortie de gaz-déchet (332) de l'unité de production d'oxygène (3), et par ailleurs à un conduit de jonction (120),
▪ des seconds moyens de pilotage (15) pilotant le dispositif de distribution de gaz (100) pour opérer une connexion fluidique entre le conduit de jonction (120) et l'une desdites sortie d'oxygène (320), entrée d'admission d'air (331) et sortie de gaz-déchet (332) de l'unité de production d'oxygène (3),
▪ des moyens de réception de cartouche (140) pour recevoir une cartouche d'O₂ (5) munie de deux valves (51, 52) contrôlant les flux gazeux entrant et sortant de la cartouche d'O₂ (5), le dispositif de distribution de gaz (100) étant relié fluidiquement à la cartouche d'O₂ (5) via le conduit de jonction (120) lorsqu'une cartouche d'O₂ (5) est positionnée dans les moyens de réception de cartouche (140), et
▪ un port de liaison à l'atmosphère (123a) en communication fluidique avec l'atmosphère ambiante (A).

2. Installation selon la revendication 1, **caractérisée en ce que** le dispositif de distribution de gaz (100) comprend une vanne rotative (110).

3. Installation selon l'une des revendications 1 ou 2, **caractérisée en ce que** le dispositif de distribution de gaz (100) comprend :
- une première entrée (100.1) reliée fluidiquement à la sortie d'oxygène (320) de l'unité de production d'oxygène (3),
- une deuxième entrée (100.2) reliée fluidiquement à l'entrée d'admission d'air (331) de l'unité de production d'oxygène (3),
- une troisième entrée (100.3) reliée fluidiquement à la sortie de gaz-déchet (332), et
- une quatrième entrée (100.4) reliée fluidiquement au conduit de jonction (120).

4. Installation selon la revendication 1, **caractérisée en ce que** les moyens de réception de cartouche (140) comprennent un logement à cartouche (130) pour loger la cartouche d'O₂ (5).

5. Installation selon la revendication 4, **caractérisée en ce que** les moyens de réception de cartouche (140) comprennent des moyens de maintien de cartouche (121, 122) pour maintenir la cartouche d'O₂ (5) dans le logement à cartouche (130).

6. Installation selon la revendication 5, **caractérisée en ce que** l'unité de remplissage (1) comprend des moyens de déplacement (160) coopérant avec les moyens de maintien de cartouche (121, 122) pour opérer un déplacement translatif de la cartouche d'O₂ (5) dans le logement à cartouche (130).

7. Installation selon les revendications 5 ou 6, **caractérisée en ce que** les moyens de maintien de cartouche (121, 122) comprennent un premier support (121) et un second support (122) agencés de part et d'autre du logement à cartouche (130), servant à maintenir mécaniquement la cartouche d'O₂ (5) en position dans le logement à cartouche (130).

8. Installation selon la revendication 1, **caractérisée en ce que** ledit au moins un module de séparation électrochimique (316) de l'unité de production d'oxygène (3) comprend une ou plusieurs membranes céramiques, de préférence une ou plusieurs membranes céramiques dopées par un ou des électrolytes.

9. Installation selon la revendication 1, **caractérisée en ce que** l'unité de production d'oxygène (3) comprend en outre des moyens d'aspiration de gaz (311), des moyens de chauffage de gaz (313) et/ou des moyens d'échange thermique (312).

10. Installation selon la revendication 6, **caractérisée en ce que** les moyens de déplacement (160) comprennent au moins un actionneur mécanique, de préférence un moteur linéaire.

11. Installation selon les revendications 1 et 6, **caractérisée en ce que** les moyens de déplacement (160) sont contrôlés par les seconds moyens de pilotage (15).

12. Installation selon la revendication 1, **caractérisée en ce que** les premiers et/ou seconds moyens de pilotage (35, 15) comprennent au moins une carte de commande électronique et au moins une unité de contrôle à microprocesseur.

13. Installation selon l'une des revendications 1, 6 ou 10 et la revendication 5, **caractérisée en ce que** les moyens de déplacement (160) sont configurés pour agir sur les moyens de maintien de cartouche (121, 122) pour déplacer la cartouche d'O₂ (5) et établir une connexion fluidique, au travers des valves (51, 52) de la cartouche d'O₂ (5), entre un volume interne (510) de la cartouche d'O₂ (5), le conduit de jonction (120) et le port de liaison à l'atmosphère (123a) relié à l'atmosphère ambiante (A), lorsqu'une cartouche d'O₂ (5) munie de deux valves (51, 52) est logée dans le logement à cartouche (130).

14. Installation selon la revendication 1, **caractérisée en ce que** le premier support (121) et/ou le second support (122) sont mobile en translation, lorsqu'ils est ou sont déplacés par les moyens de déplacement (160).

15. Procédé de régénération et remplissage d'une cartouche d'O₂ (5) munie de valves (51, 52) et contenant un matériau adsorbant (520), dans lequel on met en œuvre une installation selon l'une des revendications précédentes pour :
a) positionner la cartouche d'O₂ (5) dans les moyens de réception de cartouche (140),
b) maintenir la cartouche d'O₂ (5) au moyen des moyens de maintien de cartouche (121, 122),
c) opérer un déplacement des moyens de maintien de cartouche (121, 122) au moyen des moyens de déplacement (160) de manière à établir une connexion fluidique, au travers des valves (51, 52) de la cartouche d'O₂ (5), entre le volume interne (510) de la cartouche d'O₂ (5), le conduit de jonction (120) et le port de liaison à l'atmosphère (123a) relié à l'atmosphère ambiante (A),
d) régénérer le matériau adsorbant (520) contenu dans le volume interne (510) de la cartouche d'O₂ (5) avec un gaz-déchet à une température d'au moins 300°C provenant de l'unité de production d'oxygène (3),
e) refroidir le matériau adsorbant (520) contenu dans le volume interne (510) de la cartouche d'O₂ (5) avec de l'air provenant du port de liaison à l'atmosphère (123a),
f) déplacement des moyens de maintien de cartouche (121, 122) par les moyens de déplacement (160) pour interrompre toute communication fluidique entre l'une (52) des valves (51, 52) de la cartouche d'O₂ (5) et le port de liaison à l'atmosphère (123a),
g) remplir le volume interne (510) de la cartouche d'O₂ (5) avec de l'oxygène provenant de l'unité de production d'oxygène (3) jusqu'à obtenir une pression donnée, de préférence inférieure à 10 bar,
h) déplacement des moyens de maintien de cartouche (121, 122) par les moyens de déplacement (160) pour interrompre toute communication fluidique via l'autre (51) des valves (51, 52) de la cartouche d'O₂ (5), et
i) retirer la cartouche d'O₂ (5) remplie avec de l'oxygène.
